# EUROPÄISCHE PATENTANMELDUNG

(11) **EP 3 754 617 A1**
(43) Veröffentlichungstag der Anmeldung: **23.12.2020**
(21) Anmeldenummer: 19181776.6
(22) Anmeldetag: 21.06.2019
(51) Int. Cl.: G06T 19/20, G06T 7/00, A61B 5/055

(54) **VISUALISIERUNGSVERFAHREN, MRT-VERFAHREN UND VERFAHREN ZUR UNTERSTÜTZUNG EINER CHIRURGISCHEN BEHANDLUNG EINES MENSCHLICHEN GEHIRNS**

(71) Anmelder: Albert-Ludwigs-Universität Freiburg, 79098 Freiburg (DE)
(72) Erfinder: Reisert, Marco, 79104 Freiburg (DE); Coenen, Volker Arnd, 79283 Bollschweil (DE)
(74) Vertreter: Mertzlufft-Paufler, Cornelius

(57) **Zusammenfassung**

Zusammenfassend werden ein MRT-Verfahren (110) und ein Visualisierungsverfahren (100) beschrieben, bei der die darzustellende Information (7,18) in zwei Schritten (104,105) zu dem sodann visualisierten Zielvolumen (10) gemapped wird. Die Erfindung geht davon aus, dass die interessierende Metainformation (7) zunächst in einem Referenz-Datensatz (6) vorliegt. In einem ersten Schritt (105) wird mittels Bildregistrierung die Metainformation (7) in den Koordinatenraum gebracht, der der Anatomie des Patienten bzw. der Geometrie des Messobjekts (2) entspricht. Sodann wird in einem zweiten Schritt (107) mittels durch ein Traktografieverfahren (101) ermittelten Fasern (3) die Metainformation (7) an das Zielvolumen (10) gebracht und ausgegeben. Weiter werden Anwendungen der Verfahren (100,110) beschrieben, darunter auch ein Verfahren zur Unterstützung einer chirurgischen Behandlung eines menschlichen Gehirns (vgl. Fig. 2).

## Beschreibung

Die Erfindung betrifft ein Visualisierungsverfahren, wobei aus einem diffusionsgewichteten MRT-Datensatz eines Objekts mittels eines Traktografieverfahrens Fasern in einem Objektraum ermittelt werden.

Traktografieverfahren, häufig auch als *fiber tracking* bezeichnet, sind bereits seit vielen Jahren bekannt und dem Fachmann daher geläufig. Häufig werden derartige Verfahren eingesetzt, um den Verlauf von Nervenbahnen im Gehirn zu rekonstruieren, um sodann Konnektivitätsinformationen zu visualisieren. Derartige Verfahren sind darüber hinaus generell geeignet zur Darstellung von faserigem Gewebe mit anisotroper Diffusion.

Die Erfindung betrifft weiter ein MRT-Verfahren, wobei ein anatomischer MRT-Datensatz eines Objekts mittels eines MRT-Geräts aufgenommen wird und wobei ein diffusionsgewichteter MRT-Datensatz des Objekts mittels des MRT-Geräts aufgenommen wird.

Die Aufnahme anatomischer MRT-Datensätze mittels MRT-Geräten betrifft den Kernbereich der MRT-Technologie. Dem Fachmann ist daher bekannt, auf welche Weise er anatomische MRT-Datensätze aufnehmen kann. Dem Fachmann ist geläufig, dass er durch geeignete Wahl von Pulssequenzen unterschiedliche Bildkontraste erzielen kann wie beispielsweise ein T1- oder T2-Gewichtung. Auch die Protonendichte bildet einen bekannten Kontrast oder auch die diffusionsgewichtete Bildgebung. Für die vorliegende Erfindung kann daher vorausgesetzt werden, dass der Fachmann hinlänglich mit den zuvor genannten Methoden vertraut ist.

Die Erfindung betrifft ferner ein Verfahren zur Unterstützung einer chirurgischen Behandlung eines menschlichen Gehirns.

Aufgabe der Erfindung ist es, Konnektivitätsinformation von faserigem Gewebe einfach, und möglichst interaktionsfrei darzustellen. Eine weitere Aufgabe der Erfindung ist es, die neurochirurgische Planung zu verbessern.

Zur Lösung dieser Aufgabe schlägt die Erfindung die Merkmale von Anspruch 1 vor. Insbesondere wird somit erfindungsgemäß bei einem Visualisierungsverfahren der eingangs beschriebenen Art zur Lösung der genannten Aufgabe vorgeschlagen, dass in einem Referenzraum ein Referenz-Datensatz des Objekts mit ortsabhängiger Metainformation bereitgestellt wird, dass in dem Objektraum ein anatomischer MRT-Datensatz des Objekts bereitgestellt wird, dass mittels Bildregistrierung des anatomischen MRT-Datensatzes zu dem Referenz-Datensatz des Objekts die ortsabhängige Metainformation computergestützt auf ein Objekt-Ausgangsvolumen des Objektraums abgebildet wird, dass in dem Objektraum ein Zielvolumen ausgewählt wird, dass mittels der ermittelten Fasern die ortsabhängige Metainformation des Objekt-Ausgangsvolumens dem Zielvolumen zugeordnet wird und dass das Zielvolumen mit der zugeordneten ortsabhängigen Metainformation optisch dargestellt wird.

Die Lösung sieht somit anschaulich ausgedrückt vor, dass die darzustellende Information in zwei Schritten zu dem sodann visualisierten Zielvolumen gebracht wird. Die Erfindung geht davon aus, dass die interessierende Metainformation zunächst in einem Referenz-Datensatz vorliegt. In einem ersten Schritt wird mittels Bildregistrierung die Metainformation in den Koordinatenraum gebracht, der der Anatomie des Patienten bzw. der Geometrie des Objekts entspricht. Sodann wird in einem zweiten Schritt mittels der ermittelten Fasern die Metainformation an das Zielvolumen gebracht. Hierbei wird die Metainformation bevorzugt verarbeitet dem Zielvolumen zugeordnet. Eine verarbeitete Zuordnung wird der übliche Anwendungsfall sein. Weiter unten werden vorteilhafte Verarbeitungsmöglichkeiten genauer beschrieben.

Ein großer Vorteil der vorgeschlagenen Lösung besteht darin, dass das Zielvolumen unmittelbar die interessierende Konnektivitätsinformation enthält, so dass eine interaktionsfreie, hochaufgelöste und einfache Darstellung der interessierenden Information ermöglicht wird. Auch wird hierdurch die interessierende Information für eine weitere Verarbeitung sehr einfach verfügbar gemacht.

Die vorgeschlagene technische Lösung hat daher auch in der Anwendung eine Vielzahl praktischer Implikationen, insbesondere in der Medizin. Die Lösung ermöglicht beispielsweise eine erleichterte und genauere neurochirurgische Planung von Eingriffen, eine risikoärmere Durchführung von Ablationsverfahren im Rahmen funktioneller Eingriffe, einen besseren Schutz eloquenter Areale bei Tumorsektionen oder eine genauere Eingrenzung der wirksamen Hirnregion bei einer tiefen Hirnstimulation. Die Erfindung ermöglicht daher eine Vielzahl von Anwendungen. Einigen der in dieser Anmeldung beschriebenen Anwendungen bzw. deren Umsetzung kommt ihrerseits eine eigenständige erfinderische Qualität zu.

Bei dem Objektraum handelt es sich um den Raum, der von dem Objekt aufgespannt wird. Gewisse Verzerrungen zu der tatsächlichen Objektgeometrie können auftreten, da bei der Aufnahme des diffusionsgewichteten MRT-Datensatzes Abweichungen zu der tatsächlichen Geometrie auftreten können, beispielsweise infolge von räumlichen oder zeitlichen Imperfektionen der bei der Bildakquisition geschalteten Gradientenfelder. Entsprechendes gilt für die Aufnahme des anatomischen MRT-Datensatzes.

Bevorzugt wird sichergestellt, dass der diffusionsgewichtete MRT-Datensatz nicht verzerrt ist gegenüber dem anatomischen MRT-Datensatz. Es kann daher vorteilhaft sein, wenn die Geometrien des von dem diffusionsgewichteten MRT-Datensatz repräsentierten und von dem anatomischen MRT-Datensatz jeweils repräsentierten Objekts übereinstimmen oder allenfalls geringfügig voneinander abweichen. Dies kann bereits bei der MRT-Aufnahme sichergestellt werden oder auch im Postprocessing, beispielsweise durch eine Anwendung eines Bildregistrierungsverfahrens und/oder die Bestimmung einer Transformationsfunktion zwischen den beiden MRT-Datensätzen, die insbesondere durch ein Bildregistrierungsverfahren ermittelt werden kann. Die Transformationsfunktion kann dann auch direkt auf eine Koordinateninformation der Fasern angewandt werden.

Bei der Metainformation handelt es sich bevorzugt um Metainformation über das Objekt.

Bevorzugt umfasst der Referenz-Datensatz neben der Metainformation auch anatomische Referenzinformation, die insbesondere für die Bildregistrierung verwendbar sein kann. Im Gegensatz zu der Referenzinformation wird die Metainformation regelmäßig nicht für die Bildregistrierung benötigt. Die Metainformation stellt vielmehr eine zusätzliche Information dar.

Es kann vorgesehen sein, dass die ortsabhängige Metainformation in einem Referenz-Ausgangsvolumen des Referenzraums bereitgestellt wird, wobei das Objekt-Ausgangsvolumen des Objektraums dem Referenz-Ausgangsvolumen des Referenzraums entspricht. Diese Volumina entsprechen einander, wenn sie durch die Bildregistrierung einander zugeordnet sind und aufeinander abgebildet sind.

Die optische Darstellung kann beispielsweise durch Visualisierung auf einem Ausgabegerät wie beispielsweise einem Display erfolgen.

Bei der optischen Darstellung des Zielvolumens werden die Fasern bevorzugt ausgeblendet. Mit anderen Worten werden die Fasern bevorzugt nicht optisch dargestellt. Die Fasern werden nicht benötigt, da das Zielvolumen selbst bzw. Voxel des Zielvolumens die Konnektivitätsinformation trägt bzw. tragen. Eine Darstellung der Fasern würde daher das Bild stören.

Bei einer vorteilhaften Ausgestaltung des Verfahrens kann vorgesehen sein, dass bei der Zuordnung der Metainformation von dem Objekt-Ausgangsvolumen zu dem Zielvolumen die Metainformation entlang der Fasern zu einem Informationswert verarbeitet wird. Bevorzugt erfolgt diese Verarbeitung durch gewichtete Aufaddierung. Es werden daher Metainformationen entlang einer einzelnen Faser in gewichteter Weise aufgesammelt und zu einem Informationswert verarbeitet.

Gleiches gilt für die übrigen Fasern. Wie weiter unten noch näher dargelegt wird, kann es sich als vorteilhaft erweisen, wenn eine Verarbeitung von von mehreren Fasern aufgesammelter Metainformation zu einem gemeinsamen Informationswert erfolgt.

Der Informationswert kann eine einzelne oder mehr als eine Information darstellen. Beispielsweise kann es sich bei dem Informationswert um ein Skalar oder um einen Vektor handeln.

Die Wichtung kann je nach Anwendungsfall unterschiedlich sein. Ist die Konnektivitätsinformation entlang der Faser im Objekt-Ausgangsvolumen gleichermaßen wichtig, kann eine gleichmäßige Gewichtung zweckmäßig sein. Soll einer entfernter gelegenen Information ein größeres Gewicht beigemessen werden, kann eine Wichtung sinnvoll sein, welche mit der Entfernung zum Zielort ansteigt. Ist lokalere Information von größerem Interesse, kann es sinnvoll sein, die Wichtung in der Nähe des Zielorts stärker zu gewichten. Eine im mathematischen Sinn stetige Wichtung entlang der Fasern kann sich positiv auf die Stabilität des Visualisierungsverfahrens auswirken.

Andererseits kann gerade das Faserende im Objekt-Ausgangsvolumen eine sehr verlässliche Metainformation liefern. So kann es sich als besonders vorteilhaft erweisen, wenn die Metainformation an einem in dem Objekt-Ausgangsvolumen endenden Faserende an einen Zielort des Zielvolumens geschrieben wird, welcher durch ein weiteres Faserende definiert ist.

Bei einer weiteren vorteilhaften Ausgestaltung des Verfahrens kann vorgesehen sein, dass zur Zuordnung von Metainformation zu einem Zielort im Zielvolumen die Metainformation entlang mehrerer Fasern zu einem Informationswert verarbeitet wird. Die Verarbeitung erfolgt bevorzugt durch Aufaddierung. Derartige Ausgestaltungen können zu sehr verlässlichen und stabilen Visualisierungen führen. So kann es sinnvoll sein, die Statistik zu verbessern, indem zu jedem Zielort eine Vielzahl von Fasern berücksichtigt wird. So kann es beispielsweise sein, dass, wie weiter unten dargelegt, ein Traktografieverfahren so ausgestaltet werden kann, dass es lediglich eine Wahrscheinlichkeitsverteilung für unterschiedliche Richtungen der Fasern liefert. Eine einzelne Faser an jedem Zielort würde daher zu ungenauen Ergebnissen führen, es sei denn die Zielorte liegen sehr nah beieinander.

Üblicherweise wird es sinnvoll sein, neben dem diffusionsgewichteten MRT-Datensatz einen separaten anatomischen MRT-Datensatz aufzunehmen und/oder bereitzustellen. So können MRT-Datensätze aufgrund des verwendeten Sequenzprotokolls mit unterschiedlichem Bildkontrast verfügbar sein, die besser geeignet sind für die Bildregistrierung als der diffusionsgewichtete MRT-Datensatz. Für zeitkritische Anwendungen oder zur Senkung von Kosten kann es sich jedoch als vorteilhaft erweisen, wenn der anatomische MRT-Datensatz der diffusionsgewichtete MRT-Datensatz ist. In diesem Fall braucht nämlich lediglich ein einzelner MRT-Datensatz bereitgestellt bzw. aufgenommen zu werden.

Häufig wird Metainformation im Zielvolumen nicht verfügbar sein und aus dem Objekt-Ausgangsvolumen zuzuordnen sein. Aus diesem und anderen Gründen kann es daher vorteilhaft sein, wenn sich das Zielvolumen zumindest teilweise außerhalb des Objekt-Ausgangsvolumen befindet. Bevorzugt sind Objekt-Ausgangsvolumen und Zielvolumen disjunkt.

Bei einer weiteren vorteilhaften Ausgestaltung des Verfahrens kann vorgesehen sein, dass der ortsabhängigen Metainformation Farbwerte zugeordnet werden. Alternativ oder zusätzlich kann vorgesehen sein, dass das Zielvolumen optisch dargestellt wird, indem Farbwerte auf einem optischen Ausgabegerät wie etwa einem Display dargestellt werden. Der Farbwert kann beispielsweise durch einen reellwertigen dreidimensionalen Vektor dargestellt sein. Der Vektor kann seinerseits eindimensional parametrisiert sein. Bevorzugt werden die dargestellten Farbwerte durch Verarbeitung von der ortsabhängigen Metainformation zugeordneten Farbwerten ermittelt. Die Verarbeitung erfolgt bevorzugt durch eine gewichtete Aufaddierung von Farbwerten. Die Verwendung von Farbwerten führt zu einer besonders einfachen Umsetzung der Visualisierung. Außerdem fördern Farbwerte die Wahrnehmbarkeit der an den Anwender durch die optische Darstellung zu transportierende Information.

Eine besonders einfache Verarbeitung der Metainformation wird ermöglichst und besonders verlässliche Visualisierungen können erzielt werden, wenn die Metainformation reellwertig ist, also durch eine reellwertige Zahl oder einen reellwertiges Zahlenvektor repräsentiert wird. Bevorzugt ist die Metainformation im Ort und/oder in Bezug auf einen Parametrisierungsparameter durch eine stetige und vorzugsweise auch differenzierbare Funktion gegeben. Eine gleichsam bevorzugte Variante der Erfindung ist gegeben, wenn die Metainformation im Vergleich zum abgedeckten Zahlenraum lokal lediglich geringfügig variiert. Ein geringfügiges lokales Variieren kann beispielsweise dadurch charakterisiert sein, dass die Metainformation auf einer Distanz, die der räumlichen Auflösung des diffusionsgewichteten MRT-Datensatzes entspricht, höchstens 10%, besonders bevorzugt höchstens 5%, des von der Metainformation insgesamt abgedeckten Zahlenintervalls variiert.

Für eine Vielzahl medizinischer Anwendungen kann es vorteilhaft sein, wenn es einen Vorzugsgradienten gibt. Bevorzugt ist dieser entlang einer Hauptachse gerichtet ist. Weiter bevorzugt ist der Vorzugsgradient ein frontal-okzipitaler Gradient oder ein lateral-medialer Gradient. Bei letzterem ist der Vorzugsgradient jeweils zur Mitte hin oder von dieser weg gerichtet. Ein Vorzugsgradient kann dadurch gekennzeichnet sein, dass der Gradient zwar lokal auch signifikant von der Vorzugsrichtung abweichen kann, dass allerdings über eine größere Strecke die Metainformation lediglich entlang der Vorzugsrichtung signifikant ändert. Das Vorhandensein eines Vorzugsgradienten ist besonders vorteilhaft, wenn die Metainformation durch Farbwerte gegeben ist.

Interessante Anwendungen werden ermöglicht, wenn die ortsabhängige Metainformation eine funktionelle Eigenschaft des Objekts an dem jeweiligen Ort beschreibt. Beispielsweise kann die Metainformation eine kortikale Gehirnfunktion beschreiben.

Medizinische Anwendungen werden ermöglicht, wenn das Objekt ein menschliches Gehirn ist. Bevorzugt ist das Objekt-Ausgangsvolumen der Kortex oder ein Bereich des Kortex und das Zielvolumen ein Bereich außerhalb des Kortex wie beispielsweise der Thalamus.

Bei einer weiteren vorteilhaften Ausgestaltung des Verfahrens kann vorgesehen sein, dass bei der Bildregistrierung ein erster Satz von Voxeln mit der ortsabhängigen Metainformation verknüpft wird, dass zur Auswahl des Zielvolumens ein zweiter Satz von Voxeln ausgewählt wird, dass zur Zuordnung der Metainformation zum Zielvolumen
- zu Voxeln des zweiten Satzes mittels der ermittelten Fasern jeweils ein oder mehrere Voxel des ersten Satzes computergestützt zugeordnet werden und
- aus der mit dem einen oder den mehreren zugeordneten Voxeln des ersten Satzes verknüpften ortsabhängigen Metainformation ein Informationswert ermittelt wird, der mit dem jeweiligen Voxel des zweiten Satzes verknüpft wird.

Ein solches Verfahren hat den Vorteil, dass es zu einer einfach umsetzbaren, schnellen, stabilen und zuverlässigen Visualisierung führt, die zugleich für den Anwender zur raschen Erfassung von relevanten Informationen führt.

Zudem ermöglicht die zuvor genannte Erfindungsvariante eine Ausgestaltung, bei der die Voxel des zweiten Satzes kleiner sind als die Voxel des ersten Satzes. Dies führt zu einer Visualisierung mit einer höheren Bildauflösung als sie der anatomische und/oder diffusionsgewichtete MRT-Datensatz aufweist. Ermöglicht wird dies durch die Verwendung eines Traktografieverfahrens.

Beispielsweise kann bei einer Ausgestaltung des Verfahrens vorgesehen sein, dass im Zielvolumen Koordinaten vorgegeben werden, die Seed-Punkte für die Ermittlung von Fasern mittels des Traktografieverfahrens bilden. Bevorzugt sind die Koordinaten durch Mittelpunkte der Voxel des zweiten Satzes definiert. Bevorzugt sind ferner die Koordinaten durch Zielorte im Zielvolumen welchen die Metainformation zugeordnet wird, vorgegeben. Derartige Ausgestaltungen können durch die Wahl eng beieinander liegender Koordinaten zu sehr hohen Bildauflösungen der resultierenden Visualisierung führen.

Es hat sich herausgestellt, dass die Qualität der Visualisierung von der Wahl des Traktografieverfahrens erheblich beeinflusst werden kann. Eine hohe Bildqualität kann erreicht werden, wenn das Traktografieverfahren ein regularisiertes Verfahren ist, beispielsweise ein regularisiertes constrained spherical deconvolution Verfahren.

Eine besonders hohe Qualität kann bei der Visualisierung erzielt werden, wenn vorgesehen ist, dass das Traktografieverfahren ein zweischrittiges Verfahren ist, wobei in einem ersten Schritt zunächst eine Faserorientierungsverteilung berechnet wird und wobei aus der Faserorientierungsverteilung sodann ein Satz von Fasern generiert wird. Bevorzugt kann vorgesehen sein, dass das Traktografieverfahren eine zweifache Fasergenerierung umfasst, wobei zunächst aus einem vorherigen Satz von generierten Fasern die Faserorientierungsverteilung berechnet wird. Besonders bevorzugt übersteigt die Anzahl der Fasern des nachfolgenden Satzes die Anzahl der Fasern des vorherigen Satzes um ein Mehrfaches. Die Zweischrittigkeit des Traktografieverfahrens kann zu einer verbesserten Visualisierungsqualität führen, da durch die in einem Zwischenschritt erzeugte Faserorientierungsverteilung die Möglichkeit geschaffen wird, eine Vielzahl von Fasern zu generieren, um so die Statistik zu verbessern. Hierdurch werden statistisch bedingte Variationen in der Visualisierung minimiert und hierdurch deren Qualität verbessert.

Zur Lösung der genannten Aufgabe sind erfindungsgemäß die Merkmale des auf ein MRT-Verfahren gerichteten nebengeordneten Anspruchs vorgesehen. Insbesondere wird zur Lösung der genannten Aufgabe somit erfindungsgemäß bei einem MRT-Verfahren der eingangs beschriebenen Art vorgeschlagen, dass auf die aufgenommenen Daten ein Visualisierungsverfahren angewandt wird, welches erfindungsgemäß, insbesondere wie zuvor beschrieben und/oder nach einem der auf ein Visualisierungsverfahren gerichteten Schutzansprüche, ausgebildet ist. Ein solches MRT-Verfahren weist Vorteile auf, die den Vorteilen entsprechen, welche oben im Zusammenhang mit einem erfindungsgemäßen Visualisierungsverfahren beschrieben sind.

Zur Lösung der genannten Aufgabe sind erfindungsgemäß die Merkmale des auf ein Verfahren zur Unterstützung einer chirurgischen Behandlung eines menschlichen Gehirns gerichteten nebengeordneten Anspruchs vorgesehen. Insbesondere wird zur Lösung der genannten Aufgabe somit erfindungsgemäß bei einem solchen Verfahren vorgeschlagen, dass ein MRT-Verfahren, das erfindungsgemäß, insbesondere wie zuvor beschrieben und/oder nach einem der auf ein MRT-Verfahren gerichteten Schutzansprüche, ausgebildet ist, angewendet wird, wobei das Objekt-Ausgangsvolumen ein kortikales Volumen ist, wobei das Zielvolumen ein nicht-kortikales Volumen ist und wobei die ortsabhängige Metainformation eine Information über die Funktion des jeweils betroffenen Hirnareals bildet. Durch ein derartiges Verfahren können Nebenwirkungen eines chirurgischen Eingriffs besser abgeschätzt und vermieden werden.

Die Abkürzung "MRT" wird im Rahmen dieser Erfindungsbeschreibung als Abkürzung für "Magnetresonanztomographie" verwendet. Der Begriff "Bild" wird im Rahmen diese Erfindung weit verwendet und umfasst insbesondere auch dreidimensionale Bilder. Eine Bildregistrierung ist daher beispielsweise nicht auf die Registrierung von 2D-Bildern beschränkt, sondern umfasst auch die Registrierung von volumetrischen Datensätzen.

Die Erfindung wird nun anhand einiger weniger Ausführungsbeispiele näher beschrieben, ist jedoch nicht auf diese wenigen Ausführungsbeispiele beschränkt. Weitere Erfindungsvarianten und Ausführungsbeispiele ergeben sich durch Kombination der Merkmale einzelner oder mehrerer Schutzansprüche untereinander und/oder mit einzelnen oder mehreren Merkmalen der Ausführungsbeispiele und/oder der zuvor beschriebenen Varianten erfindungsgemäßer Vorrichtungen und Verfahren.

Es zeigt:
- Fig. 1: einen Messaufbau zur Durchführung eines erfindungsgemäßen MRT-Verfahrens,
- Fig. 2: eine Darstellung zur Erläuterung eines Ausführungsbeispiels eines erfindungsgemäßen Visualisierungsverfahrens,
- Fig. 3: ein Flussdiagramm zu dem in Fig. 2 illustrierten Visualisierungsverfahren und ein darauf aufbauendes Ausführungsbeispiel eines erfindungsgemäßen MRT-Verfahrens.

Bei der nachfolgenden Beschreibung verschiedener Ausführungsbeispiele der Erfindung erhalten in ihrer Funktion übereinstimmende Elemente auch bei abweichender Gestaltung oder Formgebung übereinstimmende Bezugszahlen.

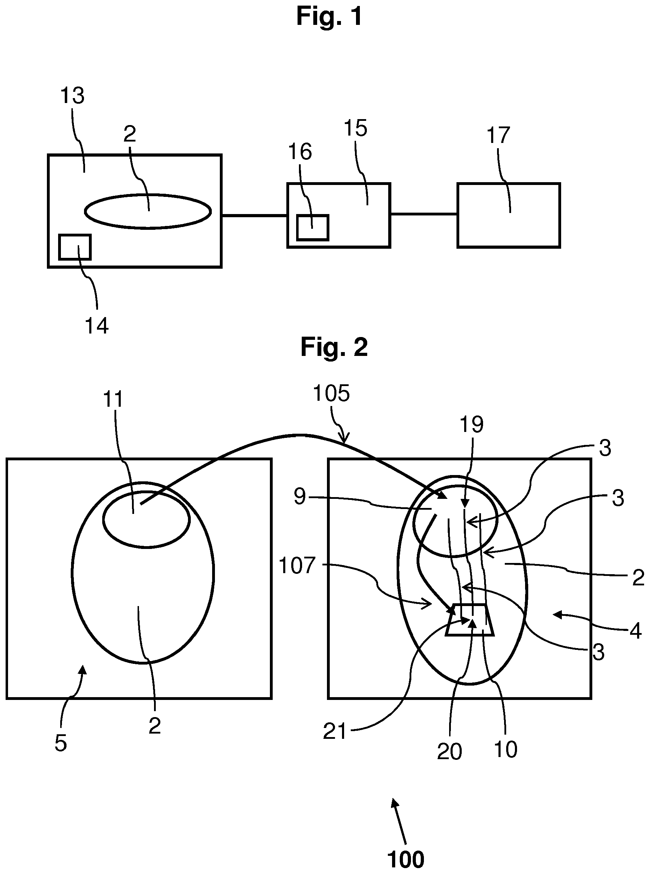

Der in Fig. 1 gezeigte Messaufbau ist zur Ausführung eines erfindungsgemäßen MRT-Verfahrens 110 geeignet, wie insbesondere das weiter unten näher dargestellte Ausführungsbeispiel eines solchen MRT-Verfahrens 110 illustriert. Der in Fig. 1 gezeigte Messaufbau umfasst ein handelsübliches MRT-Gerät 13, mit dem ein Objekt 2 ausgemessen werden kann. Es kann sich bei dem MRT-Gerät 13 insbesondere um einen MRT-Scanner für die Ganzkörper-Humanbildgebung handeln. Es kann sich jedoch auch um ein beliebiges anderes MRT-Gerät 13 handeln. Das MRT-Gerät 13 weist die üblichen Komponenten auf wie einen Hauptmagneten von beispielsweise 1.5 Tesla, eine Hochfrequenz-Sendespule zur Anregung der Spins im Objekt 2 und eine entsprechende Hochfrequenz-Empfangsspule sowie drei orthogonale Gradienten und Shimspulen. Ferner hat das MRT-Gerät 13 eine Steuereinheit zum Steuern der verschiedenen Komponenten und Applizieren geeigneter Pulssequenzen. Das MRT-Gerät 13 weist auch eine Eingabeschnittstelle 14 auf, um das MRT-Gerät 13 wie gewünscht ansteuern zu können.

Die mit dem MRT-Gerät 13 aufgenommenen MRT-Datensätze 1, 8 werden sodann an eine einen Speicher 16 aufweisende Recheneinheit 15 gesendet und in dem Speicher 16 abgespeichert. In dem Speicher 16 ist ferner ein Programm hinterlegt, mit dem wesentliche Schritte eines erfindungsgemäßen Visualisierungsverfahrens 100 ausführbar sein, wie beispielsweise entsprechende Schritte des weiter unten näher dargelegten Visualisierungsverfahrens 100. Das Ergebnis der Berechnung wird sodann an ein Ausgabegerät 17 gesendet, wo es auf einem Bildschirm angezeigt wird.

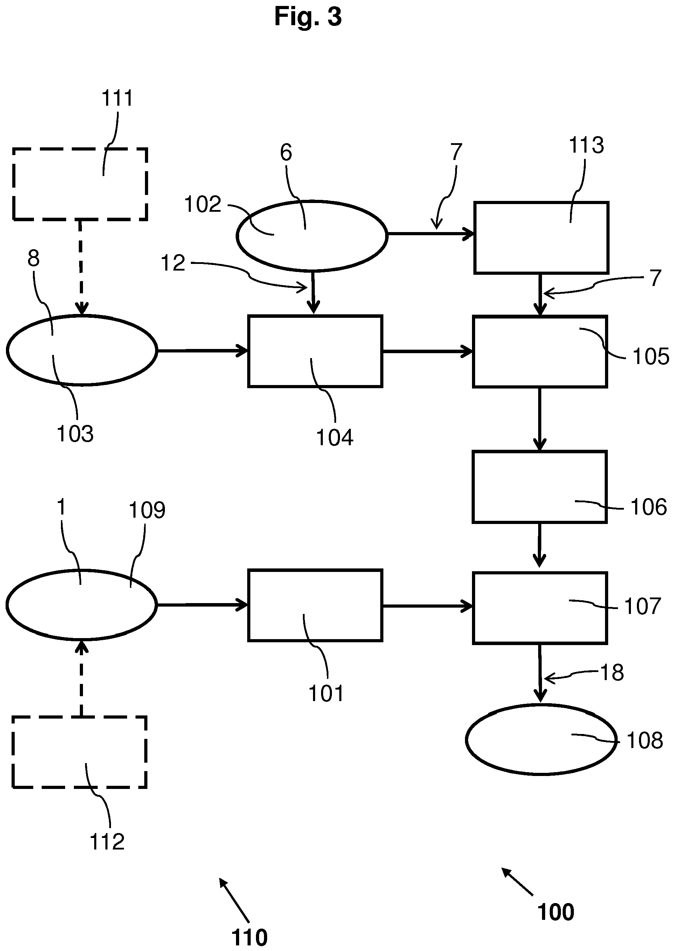

Fig. 3 zeigt ein vorteilhaftes Beispiel eines erfindungsgemäßen Visualisierungsverfahrens 100 und Beispiel eines hierauf aufbauenden erfindungsgemäßen MRT-Verfahrens 110. Fig. 2 dient in erster Linie der Erläuterung des Visualisierungsverfahrens 100. Fig. 2 und Fig. 3 werden im Folgenden gemeinsam beschrieben.

Für die Durchführung des MRT-Verfahrens 110 werden zunächst zwei MRT-Datensätze 1,8 aufgenommen. In Verfahrensschritt 111 wird ein anatomischer MRT-Datensatz 8 aufgenommen, wie beispielsweise ein Tl-gewichtetes 3D-Bild des Objekts 2. Ein solches kann beispielsweise mittels einer RARE-Seqeuenz (rapid acquisition with Relaxation Enhancement), auch bekannt unter dem Namen TSE (turbo spin echo) oder FSE (fast spin echo) aufgenommen werden. In Verfahrensschritt 112 wird ein diffusionsgewichteter MRT-Datensatz 1 aufgenommen, beispielsweise unter Anwendung einer HARDI-Sequenz (high angular resolution diffusion imaging). Die Reihenfolge der Aufnahmen sind austauschbar.

Nachdem diese MRT-Datensätze 1, 8 im Speicher 16 der Recheneinheit 15 abgelegt worden sind, decken sich die weiteren Verfahrensschritte des MRT-Verfahrens 110 mit den als nächstes beschriebenen Verfahrensschritten des erfindungsgemäßen Visualisierungsverfahrens 100.

In Schritt 102 wird ein Referenz-Datensatz 6 bereitgestellt. Hierbei kann ein eigens erstellter Referenz-Datensatz 6 verwendet werden. Es gibt allerdings auch allgemein anerkannte Referenz-Datensätze 6, welche beispielsweise im Rahmen des *Human Connectome Projects* verfügbar gemacht worden sind. Der Referenz-Datensatz 6 kann auch aus einer Verarbeitung eines allgemein anerkannten Referenz-Datensatzes 6 abgeleitet sein. Der Referenz-Datensatz 6 umfasst zum einen anatomische Referenzinformation 12, welche Informationen über die Geometrie des Referenzobjekts 2 enthält und somit für die Bildregistrierung benötigt werden. Das von dem Referenz-Datensatz 6 repräsentierte Objekt 2 stellt ein Koordinatensystem bereit, das einen Referenzraum 5 aufspannt. Das Objekt 2 des Referenz-Datensatzes 6 kann beispielsweise ein Modell eines Gehirns sein.

Der Referenz-Datensatz 6 umfasst ferner zusätzliche Metainformation 7. In dem hier beschriebenen Ausführungsbeispiel wird die Metainformation 7 durch Farbwerte dargestellt, die ihrerseits durch reellwertige Vektoren dargestellt werden. Die Farbwerte bilden eine Farbverteilung mit einem okzipital-frontalen Gradienten, beispielsweise mit einem Farbverlauf von grün über hellblau, blau, lila, rosa und rot. Ferner ist ein Farbverteilung mit einem lateral-medialen Gradienten bereitstellbar, beispielsweise mit einem Farbverlauf von orange über gelb, grün, hellblau, blau und wieder hellblau, blau, grün, gelb und orange.

Die Farbverteilung ist parametrisiert. Die Farbverteilung ist sowohl in Bezug auf die räumlichen Koordinaten wie auch in Bezug auf einen Parametrisierungsparameter im Ort stetig und differenzierbar und weist einen weichen Verlauf auf, bei dem zwischen benachbarten Punkten keine Farben übersprungen werden. Benachbarte Farben können ebenfalls weich abgegrenzt sein, sie können allerdings auch deutlicher definierte Kanten aufweisen.

Die Metainformation 7 bildet in dem hier beschriebenen Ausführungsbeispiel über die Farbverteilung ein Modell der funktionellen Struktur des Kortex ab. Hierbei sind gleiche Farben gleichen oder gleichartigen Funktionen zugeordnet. Das Modell der funktionellen Struktur ist bevorzugt von einem allgemein anerkannten Hirnmodell abgeleitet.

In Schritt 113 wird sodann ein Referenz-Ausgangsvolumen 11 festgelegt, wie beispielsweise der Kortex, und die Metainformation 7 wird in diesem Referenz-Ausgangsvolumen 11 bereitgestellt.

Parallel wird in Schritt 103 ein anatomischer MRT-Datensatz 8 bereitgestellt. Hierfür kann insbesondere zunächst einen MRT-Aufnahme wie zuvor beschrieben durchgeführt werden oder durchgeführt worden sein.

Der anatomische MRT-Datensatz 8 ist mit einer bestimmten Bildauflösung aufgenommen worden und bildet ein Gitter von Voxeln. Diese Voxel können einen ersten Satz von Voxeln vorgegeben. Der erste Satz von Voxeln kann jedoch auch von dem Voxelgitter des anatomischen MRT-Datensatzes 8 abweichen.

Der anatomische MRT-Datensatz 8 repräsentiert das Objekt 2 in einem Objektraum 4. Die Geometrie und Anatomie des Objekts 2 unterscheidet sich von der Geometrie und Anatomie des Objekts 2 im Referenzraum 5, da sie eine individuelle Eigenschaft des Objekts 2 darstellt.

In Schritt 104 werden sodann das Objekt 2 im Objektraum 4 auf das Objekt 2 im Referenzraum 5 aufeinander registriert. Dies erfolgt mittels eines Bildregistrierungsverfahren. Dem Fachmann sind geeignete Bildregistrierungsverfahren geläufig. Für die Bildregistrierung wird die Metainformation 7 nicht verwendet, sondern lediglich die anatomische Referenzinformation 12 des Referenz-Datensatzes 6.

Nach oder im Zuge der Bildregistrierung wird sodann in Schritt 105 die Metainformation 7 von dem Referenz-Ausgangsvolumen 11 auf das entsprechende Volumen im Objektraum 4, welches hier als Objekt-Ausgangsvolumen 9 bezeichnet wird, gemapped.

In Schritt 106 wird sodann oder bereits zuvor ein Zielvolumen 10 in dem Objektraum 4 festgelegt. Hierbei kann es sich beispielsweise um ein Hirnareal handeln, in dem eine chirurgische Behandlung durchgeführt werden soll. Ein solches Hirnareal kann beispielsweise der Thalamus sein.

In Schritt 107 wird die bereits in den Objektraum 4 gebrachte Metainformation 7 dem Zielvolumen 10 zugeordnet. Die Zweischrittigkeit des Visualisierungsverfahrens wird in Fig. 2 verdeutlicht. In einem ersten Schritt 105 wird die Metainformation 7 von dem Referenzraum 5 in den Objektraum 4 gebracht. In einem zweiten Schritt 107 wird sodann die Metainformation 7 dem Zielvolumen 10 zugeordnet.

Dies erfolgt, indem zunächst in Schritt 109 ein diffusionsgewichteter MRT-Datensatz 1 bereitgestellt wird. Aus diesem werden sodann mittels eines Traktografieverfahrens Fasern 3 ermittelt. Das Traktografieverfahren 101 ist hierbei wie bereits weiter oben genauer beschrieben zweischrittig ausgebildet. In einem ersten Schritt wird zunächst vorzugsweise mittels eines regularisierten Traktorgrafieverfahrens und/oder mittels eines globalen Ansatzes eine Faserorientierungsverteilung ermittelt. Es wird im Zielvolumen 10 ein zweiter Satz von Voxeln vorgegeben. Die Voxelgröße des zweiten Satzes ist hierbei beispielsweise um einen Faktor 4 entlang jeder Dimension geringer gewählt als die Voxelgröße des ersten Satzes. Zu jedem Voxel des zweiten Satzes wird ein Faserorientierungsverteilung ermittelt. Sodann wird ausgehend von jedem Voxel des zweiten Satzes jeweils eine Vielzahl von Fasern 3 generiert, die der Faserorientierungsverteilung des Voxels entsprechend verteilt sind. Hierzu kann beispielsweise eine Zufallszahl mit einer von der Faserorientierungsverteilung vorgegebenen Wahrscheinlichkeit gezogen werden.

Die Fasern 3 werden sodann verwendet, um die Metainformation 7 dem Zielvolumen 10 zuzuordnen. Dies ist möglich, da der geometrische Verlauf der Fasern 3 abgelegt und daher bekannt ist. Beispielsweise können die Fasern 3 Polygonzüge bilden, deren Koordinaten bekannt sind. Ist sichergestellt, dass die Koordinaten der Fasern 3 und die Ortsinformation der in den Objekt-Ausgangsvolumen 9 gebrachten Metainformation 7 übereinstimmen oder allenfalls geringfügig voneinander abweichen, so können die Fasern 3 eine verlässliche Zuordnung von Metainformation 7 im Objekt-Ausgangsvolumen 9 und Zielvolumen 10 bewirken.

Hierbei kann die Metainformation 7 in der zuvor beschriebenen Weise zu einem Informationswert 18 verarbeitet werden, der an ein Voxel des zweiten Satzes geschrieben wird. In einer besonders einfachen Implementierung kann beispielsweise vorgesehen sein, dass für jedes Voxel des zweiten Satzes und jede von diesem Voxel ausgehende Faser 3 geprüft wird, ob die Faser 3 im Objekt-Ausgangsvolumen 9 endet. Gibt es dort ein Faserende 19, so wird die Metainformation 7 ausgelesen, welche dem nahestgelegenen Voxel des ersten Satzes zugeordnet ist. Es werden sodann alle Metainformationen 7, die durch reellwertige Farbwerte repräsentiert sind, aufaddiert, und an das Voxel geschrieben, von dem aus die Fasern 3 starten. Dieses Zielvoxel bildet zugleich ein weiteres Faserende 20 und einen Zielort 21 im Zielvolumen 10. Alternative Möglichkeiten der Verarbeitung sind bereits weiter oben beschrieben worden, beispielsweise unter Verwendung einer Wichtungsfunktion entlang der Fasern 3.

Nachdem die Zuordnung der Metainformation 7 zu den Voxeln des zweiten Satzes im Zielvolumen 10 erfolgt ist, werden in Schritt 108 die den Voxeln des Zielvolumens 10 zugeordneten Informationswerte 18, welche Farbwerte bilden, optisch über ein Display dargestellt.

Zusammenfassend werden ein MRT-Verfahren 110 und ein Visualisierungsverfahren 100 beschrieben, bei der die darzustellende Information 7, 18 in zwei Schritten 104, 105 zu dem sodann visualisierten Zielvolumen 10 gemapped wird. Die Erfindung geht davon aus, dass die interessierende Metainformation 7 zunächst in einem Referenz-Datensatz 6 vorliegt. In einem ersten Schritt 105 wird mittels Bildregistrierung die Metainformation 7 in den Koordinatenraum gebracht, der der Anatomie des Patienten bzw. der Geometrie des Messobjekts 2 entspricht. Sodann wird in einem zweiten Schritt 107 mittels durch ein Traktografieverfahren 101 ermittelten Fasern 3 die Metainformation 7 an das Zielvolumen 10 gebracht und ausgegeben. Weiter werden Anwendungen der Verfahren 100, 110 beschrieben, darunter auch ein Verfahren zur Unterstützung einer chirurgischen Behandlung eines menschlichen Gehirns.

### Bezugszeichenliste

- 1: diffusionsgewichteter MRT-Datensatz
- 2: Objekt
- 3: Faser
- 4: Objektraum
- 5: Referenzraum
- 6: Referenz-Datensatz
- 7: Metainformation
- 8: anatomischer MRT-Datensatz
- 9: Objekt-Ausgangsvolumen
- 10: Zielvolumen
- 11: Referenz-Ausgangsvolumen
- 12: anatomische Referenzinformation
- 13: MRT-Gerät
- 14: Eingabeschnittstelle
- 15: Recheneinheit
- 16: Speicher
- 17: Ausgabegerät
- 18: Informationswert
- 19: ein Faserende
- 20: weiteres Faserende
- 21: Zielort von 10
- 100: Visualisierungsverfahren
- 101: Traktografieverfahren
- 102: Bereitstellen von 6
- 103: Bereitstellen von 8
- 104: Bildregistrierung
- 105: Abbilden von 7 auf 9
- 106: Auswählen von 10
- 107: Zuordnung von 7 zu 10
- 108: Optische Darstellung von 10
- 109: Bereitstellen von 1
- 110: MRT-Verfahren
- 111: Aufnahme von 8
- 112: Aufnahme von 1
- 113: Bereitstellen von 7 in 11

## Patentansprüche

1. Visualisierungsverfahren (100), wobei aus einem diffusionsgewichteten MRT-Datensatz (1,8) eines Objekts (2) mittels eines Traktografieverfahrens (101) Fasern (3) in einem Objektraum (4) ermittelt werden, **dadurch gekennzeichnet, dass** in einem Referenzraum (5) ein Referenz-Datensatz (6) des Objekts (2) mit ortsabhängiger Metainformation (7) bereitgestellt wird (102), dass in dem Objektraum (4) ein anatomischer MRT-Datensatz (1,8) des Objekts (2) bereitgestellt wird (103), dass mittels Bildregistrierung (104) des anatomischen MRT-Datensatzes (1,8) zu dem Referenz-Datensatz (6) des Objekts (2) die ortsabhängige Metainformation (7) computergestützt auf ein Objekt-Ausgangsvolumen (9) des Objektraums (4) abgebildet wird (105), dass in dem Objektraum (4) ein Zielvolumen (10) ausgewählt wird (106), dass mittels der ermittelten Fasern (3) die ortsabhängige Metainformation (7) des Objekt-Ausgangsvolumens (9) dem Zielvolumen (10) zugeordnet wird (107) und dass das Zielvolumen (10) mit der zugeordneten ortsabhängigen Metainformation (7) optisch dargestellt wird (108).

2. Verfahren (100) nach dem vorangehenden Anspruch, **dadurch gekennzeichnet, dass** bei der Zuordnung der Metainformation (7) von dem Objekt-Ausgangsvolumen (9) zu dem Zielvolumen (10) die Metainformation (7) entlang der Fasern (3) zu einem Informationswert (18) verarbeitet wird, insbesondere durch gewichtete Aufaddierung, wobei besonders bevorzugt die Metainformation (7) an einem in dem Objekt-Ausgangsvolumen (9) endenden Faserende (19) an einen Zielort (21) des Zielvolumens (10) geschrieben wird, welcher durch ein weiteres Faserende (20) definiert ist.

3. Verfahren (100) nach einem der vorangehenden Ansprüche, **dadurch gekennzeichnet, dass** zur Zuordnung (107) von Metainformation (7) zu einem Zielort (21) im Zielvolumen (10) die Metainformation (7) entlang mehrerer Fasern (3) zu einem Informationswert (18) verarbeitet wird, insbesondere durch Aufaddierung.

4. Verfahren (100) nach einem der vorangehenden Ansprüche, **dadurch gekennzeichnet, dass** der anatomische MRT-Datensatz (1,8) der diffusionsgewichtete MRT-Datensatz (1,8) ist.

5. Verfahren (100) nach einem der vorangehenden Ansprüche, **dadurch gekennzeichnet, dass** sich das Zielvolumen (10) zumindest teilweise außerhalb des Objekt-Ausgangsvolumen (9) befindet, insbesondere wobei Objekt-Ausgangsvolumen (9) und Zielvolumen (10) disjunkt sind.

6. Verfahren (100) nach einem der vorangehenden Ansprüche, **dadurch gekennzeichnet, dass** der ortsabhängigen Metainformation (7) Farbwerte zugeordnet werden und/oder dass das Zielvolumen (10) optisch dargestellt wird, indem Farbwerte auf einem optischen Ausgabegerät (17) dargestellt werden (108), insbesondere wobei die dargestellten Farbwerte durch Verarbeitung wie etwa einer gewichteten Aufaddierung von der ortsabhängigen Metainformation (7) zugeordneten Farbwerten ermittelt werden.

7. Verfahren (100) nach einem der vorangehenden Ansprüche, **dadurch gekennzeichnet, dass** die Metainformation (7) reellwertig ist, insbesondere wobei die Metainformation (7) im Ort und/oder eine Parametrisierung der Metainformation (7) durch eine stetige und vorzugsweise differenzierbare Funktion gegeben ist und/oder wobei die Metainformation (7) im Vergleich zum abgedeckten Zahlenraum lokal lediglich geringfügig variiert.

8. Verfahren (100) nach dem vorangehenden Anspruch, **dadurch gekennzeichnet, dass** es einen Vorzugsgradienten gibt, insbesondere wobei der Verzugsgradient entlang einer Hauptachse gerichtet ist und/oder wobei der Vorzugsgradient ein frontal-okzipitaler Gradient oder ein lateral-medialer Gradient ist.

9. Verfahren (100) nach einem der vorangehenden Ansprüche, **dadurch gekennzeichnet, dass** die ortsabhängige Metainformation (7) eine funktionelle Eigenschaft des Objekts (2) an dem jeweiligen Ort beschreibt, insbesondere wobei die Metainformation (7) eine kortikale Gehirnfunktion beschreibt, und/oder dass das Objekt (2) ein menschliches Gehirn ist, insbesondere wobei das Objekt-Ausgangsvolumen (9) der Kortex oder ein Bereich des Kortex und das Zielvolumen (10) ein Bereich außerhalb des Kortex ist.

10. Verfahren (100) nach einem der vorangehenden Ansprüche, **dadurch gekennzeichnet, dass** bei der Bildregistrierung (104) ein erster Satz von Voxeln mit der ortsabhängigen Metainformation (7) verknüpft wird, dass zur Auswahl des Zielvolumens (10) ein zweiter Satz von Voxeln ausgewählt wird, dass zur Zuordnung (107) der Metainformation (7) zum Zielvolumen (10)
- zu Voxeln des zweiten Satzes mittels der ermittelten Fasern (3) jeweils ein oder mehrere Voxel des ersten Satzes computergestützt zugeordnet werden und
- aus der mit dem einen oder den mehreren zugeordneten Voxeln des ersten Satzes verknüpften ortsabhängigen Metainformation (7) ein Informationswert (18) ermittelt wird, der mit dem jeweiligen Voxel des zweiten Satzes verknüpft wird.

11. Verfahren (100) nach dem vorangehenden Anspruch, **dadurch gekennzeichnet, dass** die Voxel des zweiten Satzes kleiner sind als die Voxel des ersten Satzes.

12. Verfahren (100) nach einem der vorangehenden Ansprüche, **dadurch gekennzeichnet, dass** im Zielvolumen (10) Koordinaten (21) vorgegeben werden, die Seed-Punkte (21) für die Ermittlung von Fasern (3) mittels des Traktografieverfahrens (101) bilden, insbesondere wobei die Koordinaten (21) durch Mittelpunkte (21) der Voxel des zweiten Satzes definiert sind und/oder wobei die Koordinaten (21) durch Zielorte (21) im Zielvolumen (10), welchen die Metainformation (7) zugeordnet wird, vorgegeben sind.

13. Verfahren (100) nach einem der vorangehenden Ansprüche, **dadurch gekennzeichnet, dass** das Traktografieverfahren (101) eine zweifache Fasergenerierung umfasst, wobei aus einem ersten Satz von generierten Fasern (3) eine Faserorientierungsverteilung berechnet wird und wobei aus der Faserorientierungsverteilung sodann ein zweiter Satz von Fasern (3) generiert wird, insbesondere wobei die Anzahl der Fasern (3) des zweiten Satzes die Anzahl der Fasern (3) des ersten Satzes um ein Mehrfaches übersteigt.

14. MRT-Verfahren (110), wobei ein anatomischer MRT-Datensatz (1,8) eines Objekts (2) mittels eines MRT-Geräts (13) aufgenommen wird (111,112) und wobei ein diffusionsgewichteter MRT-Datensatz (1,8) des Objekts (2) mittels des MRT-Geräts (13) aufgenommen wird (111,112), **dadurch gekennzeichnet, dass** auf die aufgenommenen Daten (1,8) das Visualisierungsverfahren (100) nach einem der vorangehenden Ansprüche angewandt wird.

15. Verfahren zur Unterstützung einer chirurgischen Behandlung eines menschlichen Gehirns, **dadurch gekennzeichnet, dass** ein MRT-Verfahren (110) nach dem vorangehenden Anspruch angewandt wird, wobei das Objekt-Ausgangsvolumen (9) ein kortikales Volumen ist, wobei das Zielvolumen (10) ein nicht-kortikales Volumen ist und wobei die ortsabhängige Metainformation (7) eine Information über die Funktion des jeweils betroffenen Hirnareals bildet.
